# EUROPEAN PATENT SPECIFICATION

(11) **EP 3 595 626 B1**
(45) Date of publication and mention of the grant of the patent: **13.10.2021**
(21) Application number: 18700680.4
(22) Date of filing: 17.01.2018
(51) Int. Cl.: A61K 8/39, A61Q 11/00, A61K 8/86, A61K 8/25

(54) **ORAL CARE COMPOSITIONS**
MUNDPFLEGEZUSAMMENSETZUNGEN
COMPOSITIONS DE SOINS BUCCO-DENTAIRES

(30) Priority: 13.03.2017 EP 17160637
(43) Date of publication of application: 22.01.2020
(73) Proprietor: Unilever IP Holdings B.V., 3013 AL Rotterdam (NL); Unilever Global IP Limited, Wirral, CH62 AZD (GB)
(72) Inventor: JOINER, Andrew, Wirral Merseyside CH63 3JW (GB); LITTLEWOOD, David, Thomas, Wirral Merseyside CH63 3JW (GB); PHILPOTTS, Carole, Jane, Wirral Merseyside CH63 3JW (GB)
(74) Representative: Tansley, Sally Elizabeth
(86) International application number: PCT/EP2018/051122
(87) International publication number: WO 2018/166673

(56) References cited:
- EP-A1- 0 319 884
- WO-A1-2013/077127
- WO-A1-2015/106915
- WO-A1-2015/158637
- WO-A2-2011/056759
- DATABASE GNPD [Online] MINTEL; August 2016 (2016-08), XP002769189, Database accession no. 4236111

## Description

The present invention is concerned with oral care compositions. More particularly, the present invention is concerned with oral care compositions having low abrasivity whilst maintaining desirable cleaning properties.

Abrasives for use in oral care compositions such as dentifrices, are required to be effective in removing extrinsic stains, dental plaque and food debris which builds up on the pellicle on the surface of teeth.

In general, the efficiency of physical removal of stain, plaque and food debris can be increased by using an abrasive having increased abrasivity. However, increasing abrasivity also increases the risk that tooth surfaces may be damaged.

Accordingly, there is a continuing need for oral care compositions that demonstrate satisfactory levels of cleaning, yet are not unduly abrasive and damaging to the teeth.

EP 0 319 884 discloses that linear high molecular weight polymer selected from the group consisting of polyacrylamide derivatives decreases abrasivity and increases cleaning and stain removal.

US 4,743,274 relates to the modification of crystal morphology to provide a high cleaning, low abrading abrasive for use in oral compositions. This describes how, in the case of an abrasive which consists of calcium hydrogenphosphate anhydride (secondary calcium phosphate anhydride), reducing the average crystallite size produces a material which meets both the requirements of high tooth cleanability and low tooth surface abrasiveness.

The present application relates how oral care compositions of the invention demonstrate satisfactory levels of cleaning, yet are not unduly abrasive and damaging to the teeth. A further advantage of the oral care compositions of the invention is that they are gentle enough for use on bridgework, dentures and other forms of artificial teeth which are made from polymers much softer than natural tooth enamel, such as acrylic resin (PMMA).

### Description

The present invention provides an oral care composition comprising a silica abrasive, non-ionic surfactant comprising a polyethylene glycol ethers of fatty alcohols and a poly(ethylene oxide) polymer having a molecular weight (Mn) 300,000 or greater.

The invention further relates to the use of a poly(ethylene oxide) polymer having a molecular weight (Mn) 300,000 or greater for mitigating the abrasivity of a toothpaste.

### Detailed Description

The oral care composition comprises a nonionic surfactant comprising a polyethylene glycol ethers of fatty alcohols preferably in an amount of from 0.2 to 10% by weight based on the total weight of the composition. Polyethylene glycol ethers having from 20 to 40 ethylene oxide groups per unit. An example of such a suitable surfactant includes the group of surfactants known as Steareth surfactants such as Steareth 30. Preferably the level of non-ionic surfactant is from 0.5 to 7 wt% of the total composition, more preferably from 0.8 to 5 wt%, most preferably from 1 to 3 wt% of the total composition.

Anionic surfactants may be present in the composition but it is preferred if their level is below 0.5 wt%, more preferably 0.1 wt% of the total composition.

Although other surfactants may be present, it is preferred if they are limited to levels below 0.5 wt% of the total composition preferably less than 0.1 wt% of the total composition.

The oral care composition comprises an abrasive silica. Preferably the silica is present in an amount of from 2 to 75% wt% of the total composition, more preferably from 3 to 40 wt% of the total composition, most preferably from 3 to 25 wt% of the total composition.

The composition may comprise other abrasives but if present they should be present at lower levels than the silica abrasive. Suitable other abrasive materials include calcium carbonates, dicalcium phosphate, tricalcium phosphate, calcined alumina, sodium and potassium metaphosphate, sodium and potassium pyrophosphates, sodium trimetaphosphate, sodium hexametaphosphate, particulate hydroxyapatite and mixtures thereof.

Compositions of the invention comprise poly (ethylene oxide) polymer having a molecular weight (Mn) 300,000 or greater, more preferably a molecular weight (Mn) 800,000 or greater, most preferably a molecular weight (Mn) 2 million or greater.

The poly (ethylene oxides) employed in this invention are preferably solid, colourless, water-soluble resins.

Preferred poly (ethylene oxide) polymers are known commercially as Polyox, particularly preferred is Polyox WSR301 from the Dow Chemical Company.

The poly (ethylene oxide) polymer is preferably present at levels from 0.05 to 5 wt% of the total composition, more preferably from 0.1 to 3 wt% most preferably from 0.3 to 2 wt% of the total composition

Preferably the weight ratio of total surfactant in the composition to poly(ethylene) oxide polymer is from 20:1 to 12, more preferably from 12:1 to 1:1 most preferably from 8:1 to 2:1.

A highly preferred type of product form in the context of the present invention is a dentifrice. The term "dentifrice" denotes a formulation which are used to clean the surfaces of the oral cavity. The dentifrice is an oral composition that is not intentionally swallowed for purposes of systemic administration of therapeutic agents, but is retained in the oral cavity for a sufficient time to contact substantially all of the dental surfaces and/or mucosal tissues for purposes of oral activity. Preferably the dentifrice is suitable for application with a toothbrush and is rinsed off after use. Preferably the dentifrice is in the form of an extrudable semi-solid such as a cream, paste or gel (or mixture thereof).

The compositions of the invention, in particular the dentifrice composition will usually contain a liquid continuous phase in an amount of from 40 to 99% by weight based on the total weight of the dentifrice. Such a liquid continuous phase will typically comprise a mixture of water and polyhydric alcohol in various relative amounts, with the amount of water generally ranging from 10 to 45% by weight (based on the total weight of the dentifrice) and the amount of polyhydric alcohol generally ranging from 20 to 70% by weight (based on the total weight of the dentifrice). Typical polyhydric alcohols include humectants such as glycerol, sorbitol, polyethylene glycol, polypropylene glycol, propylene glycol, xylitol (and other edible polyhydric alcohols), hydrogenated partially hydrolyzed polysaccharides and mixtures thereof. Sorbitol is particularly preferred.

Most preferably the composition of the invention is organic polyol-based. In the context of the present invention, the term "organic polyol-based" means that the composition is not oil-based or water-based, but instead, organic polyols (as defined above) are a principal component in the composition. By "principal component" is meant that the organic polyols (as defined above) when taken together, make up a higher portion of the composition's weight than any other compound.

Furthermore, the composition, preferably the dentifrice will usually contain a binder or thickening agent in an amount of from 0.1 to 12% by weight more preferably 0.3 to 10 % by weight based on the total weight of the dentifrice. Suitable binders or thickening agents include carboxyvinyl polymers (such as polyacrylic acids cross-linked with polyallyl sucrose or polyallyl pentaerythritol), hydroxyethyl cellulose, hydroxypropyl cellulose, water soluble salts of cellulose ethers (such as sodium carboxymethyl cellulose and sodium carboxymethyl hydroxyethyl cellulose), natural gums (such as carrageenan, gum karaya, guar gum, xanthan gum, gum arabic, and gum tragacanth), finely divided silicas, hectorites, colloidal magnesium aluminium silicates and mixtures thereof.

Compositions of the present invention (such as in particular dentifrices) may also contain further optional ingredients customary in the art such as fluoride ion sources, anticalculus agents, buffers, flavouring agents, sweetening agents, colouring agents, opacifying agents, preservatives, antisensitivity agents and antimicrobial agents.

The invention is further illustrated with reference to the following, non-limiting Examples. Examples according to the invention are illustrated by a number, comparative examples are illustrated by a letter.

### EXAMPLES

The following compositions were prepared

**Table 1**

| **Chemical Name** | **Example A** | **Example B** |
|---|---|---|
| Sorbitol | 45 | 45 |
| Polyox WSR301 (MW 4 million) | 0 | 1 |
| Abrasive silica | 10 | 10 |
| Thickening silica | 7.5 | 7.5 |
| Polyethylene glycol | 5 | 5 |
| Sodium lauryl sulphate | 1.5 | 1.5 |
| Titanium dioxide | 1 | 1 |
| Sodium carboxy methyl cellulose | 0.63 | 0.63 |
| Sodium fluoride | 0.32 | 0.32 |
| Water and minors | To 100 | To 100 |

**Table 2**

| **Chemical name** | **Example C** | **Example D** |
|---|---|---|
| Calcium carbonate | 40 | 40 |
| Benzyl alcohol | 0.5 | 0.5 |
| Thickening silica | 4.7 | 4.7 |
| Sodium lauryl sulphate | 2.2 | 2.2 |
| Sorbitol | 20 | 20 |
| Trisodium phosphate | 0.5 | 0.5 |
| Sodium carboxy methyl cellulose | 0.6 | 0.6 |
| Titanium dioxide | 1 | 1 |
| Sodium monofluorophosphate | 1.11 | 1.11 |
| Polyox WSR301 (MW 4 million) | 0 | 1 |
| Water and minors | To 100 | To 100 |

**Table 3**

| | **Example E** | **Example 1** |
|---|---|---|
| **Chemical name** | % w/w | % w/w |
| Sorbitol | 65 | 65 |
| Polyethylene glycol | 1.5 | 1.5 |
| Sodium fluoride | 0.32 | 0.32 |
| Sodium saccharin | 0.2 | 0.2 |
| Trisodium phosphate | 0.5 | 0.5 |
| Polyox WSR301 (MW 4 million) | 0 | 1 |
| Poly(methylvinylether/maleic acid) | 1 | 1 |
| Thickening silica | 4.5 | 4.5 |
| Abrasive silica | 8 | 8 |
| Abrasive silica | 1.65 | 1.65 |
| Sodium carboxy methyl cellulose | 0.52 | 0.52 |
| Steareth 30 | 1.8 | 1.8 |
| Water and minors | To 100 | To 100 |

**Table 4**

| | **Example F** |
|---|---|
| **Chemical name** | **% w/w** |
| Sorbitol | 65 |
| Polyethylene glycol | 1.5 |
| Sodium fluoride | 0.32 |
| Sodium saccharin | 0.2 |
| Trisodium phosphate | 0.5 |
| Polyox WSR301 (MW 4 million) | 0 |
| Poly(methylvinylether/maleic acid) | 1 |
| Thickening silica | 4.5 |
| Abrasive silica | 8 |
| Abrasive silica | 1.65 |
| Sodium carboxy methyl cellulose | 0.52 |
| Sodium lauryl sulphate | 1.8 |
| Water and minors | To 100 |

**Table 5**

| **Chemical Name** | **Ex. G** | **Ex. 2** | **Ex. 3** | **Ex. 4** | **Ex. 5** | **Ex. 6** | **Ex. H** |
|---|---|---|---|---|---|---|---|
| Sorbitol | 65 | 65 | 65 | 65 | 65 | 65 | 65 |
| Polyox WSR301 (MW 4 million) | | 0.1 | | | | | |
| Polyox WSR301 (MW 4 million) | | | 0.5 | | | | |
| Polyox WSR301 (MW 4 million) | | | | 1.0 | | | |
| Polyox 12K (MW 1 million) | | | | | 1 | 2 | |
| Polyox N10 (MW 100,000) | | | | | | | 1 |
| Polyethylene glycol | 1.5 | 1.5 | 1.5 | 1.5 | 1.5 | 1.5 | 1.5 |
| Trisodium phosphate | 1.1 | 1.1 | 1.1 | 1.1 | 1.1 | 1.1 | 1.1 |
| Abrasive silica | 8 | 8 | 8 | 8 | 8 | 8 | 8 |
| Thickening silica | 3.1 | 3.1 | 3.1 | 3.1 | 3.1 | 3.1 | 3.1 |
| Abrasive silica | 1.7 | 1.7 | 1.7 | 1.7 | 1.7 | 1.7 | 1.7 |
| Sodium carboxy methyl cellulose | 0.6 | 0.6 | 0.6 | 0.6 | 0.6 | 0.6 | 0.6 |
| Poly(methylvinylether/maleic acid) | 1 | 1 | 1 | 1 | 1 | 1 | 1 |
| Steareth 30 | 1.8 | 1.8 | 1.8 | 1.8 | 1.8 | 1.8 | 1.8 |
| Water and minors | To 100 | To 100 | To 100 | To 100 | To 100 | To 100 | To 100 |

Molecular Weights of Polyox as follows:

| | |
|---|---|
| WSR301 | Ex Dow Chemical Company 4 million |
| 12K | Ex Dow Chemical Company 1 million |
| N10 | Ex Dow Chemical Company 100,000 |

Table 6 illustrates the enhanced decrease in RDA caused by the presence of a high molecular weight poly(ethylene oxide) polymer in a formulation comprising silica abrasive compared with a formulation comprising a carbonate abrasive.

**Table 6**

| **Formulation** | **RDA (s.e.)** |
|---|---|
| Example A | 100.42 (2.40) |
| Example B | 71.36 (2.82) |
| Example C | 174.57 (1.69) |
| Example D | 163.12 (5.54) |

The above table demonstrates that when polyox WS301 is present in a composition comprising silica abrasive the abrasivity of the formulation is significantly decreased. The effect is not so marked in a formulation comprising calcium carbonate abrasive.

**Table 7**

| **Formulation code** | **Mean RDA (s.e.)** | **PCR (s.d.)^{**}** |
|---|---|---|
| Example F | 107.41 (3.51) | -- |
| Example E | 91.90 (2.98) | 22 (10) |
| Example 1 | 48.21 (3.77) | 57 (6) |

Table 7 demonstrates that the Example according to the invention has significantly reduced abrasion together with enhanced cleaning properties.

**Table 8**

| **Toothpaste** | **Mean RDA value (S.D.)** |
|---|---|
| Example G | 110.08 (5.54) |
| Example 2 | 98.27 (2.71) |
| Example 3 | 61.04 (3.21) |
| Example 4 | 60.29 (6.36) |
| Example H | 104.51 (4.08) |
| Example 5 | 85.05 (4.76) |
| Example 6 | 86.83 (4.93) |

Molecular Weights of Polyox as follows:

| | |
|---|---|
| WSR301 | 4 million |
| 12K | 1 million |
| N10 | 100,000 |

Table 8 demonstrates the importance of molecular weight of the poly(ethylene) oxide polymer in reducing the abrasivity of a toothpaste.

## Claims

1. An oral care composition comprising a silica abrasive, non-ionic surfactant comprising a polyethylene glycol ethers of fatty alcohols and a poly(ethylene oxide) polymer having a molecular weight (Mn) 300,000 or greater.

2. An oral care composition according to claim 1 in which the poly(ethylene oxide) polymer has a molecular weight (Mn) 800,000 or greater.

3. An oral care composition according to any preceding claim in which the poly(ethylene oxide) polymer has a molecular weight (Mn) 2 million or greater.

4. An oral care composition according to any preceding claim in which the weight ratio of total surfactant to poly(ethylene oxide) polymer is from 12:1 to 1:1.

5. An oral care composition according to any preceding claim in which the level of poly(ethylene oxide) polymer is from 0.1 to 3 wt% of the total composition.

6. An oral care composition according to any preceding claim in which the level of non-ionic surfactant is from 0.5 to 7 wt% of the total composition.

7. An oral care composition according to any preceding claim in which the non-ionic surfactant is an ethoxylated surfactant having a degree of ethoxylation from 20 to 40.

8. An oral care composition according to any preceding claim in which the non-ionic surfactant is steareth 30.

9. An oral care composition according to any preceding claim in which the level of silica abrasive is from 3 to 25 wt% of the total composition.

10. An oral care composition according to any preceding claim 1 which comprises a liquid continuous phase compriisng a mixture of water and polyhydric alcohol.

11. An oral care composition according to claim 10 in which the polyhydric alcohol is sorbitol.

12. An oral care composition according to any preceding claim in which the level of polyhydric alcohol in the composition is from 20 to 70 wt% of the total composition.

13. An oral care composition according to any preceding claim which is in the form of a dentifrice.

14. Use of a poly(ethylene oxide) polymer having a molecular weight of (Mn) 300,000 or greater for mitigating the abrasivity of a toothpaste comprising : silica abrasive and a non-ionic surfactant comprising a polyethylene glycol ethers of fatty alcohols.

## Patentansprüche

1. Mundpflegezusammensetzung, umfassend einen Siliciumdioxid-Putzkörper, nichtionisches Tensid, umfassend einen Polyethylenglycolether von Fettalkoholen, und ein Poly(ethylenoxid)-Polymer mit einem Molekulargewicht (Mn) von 300.000 oder größer.

2. Mundpflegezusammensetzung nach Anspruch 1, bei der das Poly(ethylenoxid)-Polymer ein Molekulargewicht (Mn)von 800.000oder größer aufweist.

3. Mundpflegezusammensetzung nach irgendeinem vorhergehenden Anspruch, bei der das Poly(ethylenoxid)-Polymerein Molekulargewicht (Mn) von 2 Millionen oder größer aufweist.

4. Mundpflegezusammensetzung nach irgendeinem vorhergehenden Anspruch, bei der das Gewichtsverhältnisvon Gesamttensid zu Poly(ethylenoxid)-Polymer 12:1 bis 1:1 beträgt.

5. Mundpflegezusammensetzung nach irgendeinem vorhergehenden Anspruch, bei der der Gehalt an Poly(ethylenoxid)-Polymer 0,1 bis 3 Gew.-% der Gesamtzusammensetzung beträgt.

6. Mundpflegezusammensetzung nach irgendeinem vorhergehenden Anspruch, bei der der Gehalt an nichtionischem Tensid 0,5 bis 7 Gew.-% der Gesamtzusammensetzung beträgt.

7. Mundpflegezusammensetzung nach irgendeinem vorhergehenden Anspruch, bei der das nichtionischeTensid ein ethoxyliertes Tensid mit einem Ethoxylierungsgrad von 20 bis 40 ist.

8. Mundpflegezusammensetzung nach irgendeinem vorhergehenden Anspruch, bei der das nichtionischeTensid Steareth 30 ist.

9. Mundpflegezusammensetzung nach irgendeinem vorhergehenden Anspruch, bei der der Gehalt an Siliciumdioxid-Putzkörper 3 bis 25 Gew.-% der Gesamtzusammensetzung beträgt.

10. Mundpflegezusammensetzung nach irgendeinem vorhergehenden Anspruch, welche eine flüssige kontinuierliche Phase umfasst, die eine Mischung von Wasser und mehrwertigem Alkohol umfasst.

11. Mundpflegezusammensetzung nach Anspruch 10, bei der der mehrwertige Alkohol Sorbit ist.

12. Mundpflegezusammensetzung nach irgendeinem vorhergehenden Anspruch, bei der der Gehalt an mehrwertigem Alkohol in der Zusammensetzung 20 bis 70 Gew.-% der Gesamtzusammensetzung beträgt.

13. Mundpflegezusammensetzung nach irgendeinem vorhergehenden Anspruch, die in Form eines Zahnputzmittels vorliegt.

14. Verwendung eines Poly(ethylenoxid)-Polymers mit einem Molekulargewicht (Mn) von 300.000 oder größer zur Minderung der Abrasivität einer Zahnpasta,die Siliciumdioxid-Putzkörper und ein nichtionisches Tensid, umfassend einen Polyethylenglycolether von Fettalkoholen, umfasst.

## Revendications

1. Composition pour le soin oral comprenant un abrasif de silice, un tensioactif non-ionique comprenant un polyéthylène glycol éther d'alcools gras et un polymère de poly(oxyde d'éthylène) ayant une masse moléculaire (Mn) de 300 000 ou supérieure.

2. Composition pour le soin oral selon la revendication 1, dans lequel le polymère de poly(oxyde d'éthylène) présente une masse moléculaire (Mn) de 800 000 ou supérieure.

3. Composition pour le soin oral selon l'une quelconque des revendications précédentes, dans laquelle le polymère de poly(oxyde d'éthylène) présente une masse moléculaire (Mn) de 2 millions ou supérieure.

4. Composition pour le soin oral selon l'une quelconque des revendications précédentes, dans laquelle le rapport massique de tensioactif total à polymère de poly(oxyde d'éthylène) est de 12:1 à 1:1.

5. Composition pour le soin oral selon l'une quelconque des revendications précédentes, dans laquelle la teneur en polymère de poly(oxyde d'éthylène) est de 0,1 à 3 % en masse de la composition totale.

6. Composition pour le soin oral selon l'une quelconque des revendications précédentes, dans laquelle la teneur en tensioactif non-ionique est de 0,5 à 7 % en masse de la composition totale.

7. Composition pour le soin oral selon l'une quelconque des revendications précédentes, dans laquelle le tensioactif non-ionique est un tensioactif éthoxylé ayant un degré d'éthoxylation de 20 à 40.

8. Composition pour le soin oral selon l'une quelconque des revendications précédentes, dans laquelle le tensioactif non-ionique est steareth 30.

9. Composition pour le soin oral selon l'une quelconque des revendications précédentes, dans laquelle la teneur en abrasif de silice est de 3 à 25 % en masse de la composition totale.

10. Composition pour le soin oral selon l'une quelconque des revendications précédentes qui comprend une phase continue liquide comprenant un mélange d'eau et d'alcool polyvalent.

11. Composition pour le soin oral selon la revendication 10, dans laquelle l'alcool polyvalent est le sorbitol.

12. Composition pour le soin oral selon l'une quelconque des revendications précédentes, dans laquelle la teneur en alcool polyvalent dans la composition est de 20 à 70 % en masse de la composition totale.

13. Composition pour le soin oral selon l'une quelconque des revendications précédentes qui est dans la forme d'un dentifrice.

14. Utilisation d'un polymère de poly(oxyde d'éthylène) ayant une masse moléculaire de (Mn) 300 000 ou supérieure pour mitiger l'abrasivité d'une pâte dentaire comprenant un abrasif de silice et un tensioactif non ionique comprenant un polyéthylène glycol éther d'alcools gras.
